# EUROPÄISCHE PATENTSCHRIFT

(11) **EP 1 121 112 B1**
(45) Veröffentlichungstag und Bekanntmachung des Hinweises auf die Patenterteilung: **05.06.2002**
(21) Anmeldenummer: 99948972.7
(22) Anmeldetag: 09.10.1999
(51) Int. Cl.: A61K 31/165, A61K 9/12, A61P 11/06, A61P 11/08

(54) **LAGERFÄHIGES WIRKSTOFFKONZENTRAT MIT FORMOTEROL**
STABLE FORMOTEROL CONCENTRATE
CONCENTRE STABLE DE FORMOTEROL

(30) Priorität: 17.10.1998 DE 19847969; 14.12.1998 US 112380 P
(43) Veröffentlichungstag der Anmeldung: 08.08.2001
(73) Patentinhaber: Boehringer Ingelheim Pharma KG, 55218 Ingelheim am Rhein (DE)
(72) Erfinder: HOCHRAINER, Dieter, D-55411 Bingen am Rhein (DE); ZIERENBERG, Bernd, D-55411 Bingen am Rhein (DE)
(74) Vertreter: Laudien, Dieter, Dr.
(86) Internationale Anmeldenummer: EP9907581
(87) Internationale Veröffentlichungsnummer: WO0023065

(56) Entgegenhaltungen:
- WO-A-93/11747
- WO-A-97/47286
- WO-A-98/05302

## Beschreibung

Die vorliegende Erfindung betrifft ein lagerfähiges, treibgasfreies Wirkstoffkonzentrat mit Formoterol zur Verwendung in Inhalatoren zur inhalativen oder nasalen Therapie.

Formoterol ist ein aus dem Adrenalin abgeleitetes Anilid der Formel I und wird als β₂-Stimulator in der inhalativen Therapie von Lungenwegserkrankungen
insbesondere zur Behandlung von Asthma bronchiale eingesetzt. Bei Patienten mit reversiblen obstruktiven Atemwegserkrankungen wirkt Formoterol bronchodilatorisch. Bereits 1-3 Minuten nach der inhalativen Aufnahme setzt die Wirkung ein und die bronchodilatorische Wirkung ist auch nach 12 Stunden noch signifikant vorhanden. Formoterol hemmt die Freisetzung von Leukotrienen und anderen mit Entzündungsgeschehen einhergehenden Botenstoffen, wie z.B. Histaminen. Darüberhinaus kann Formoterol eine hyperglykämische Wirkung hervorrufen.

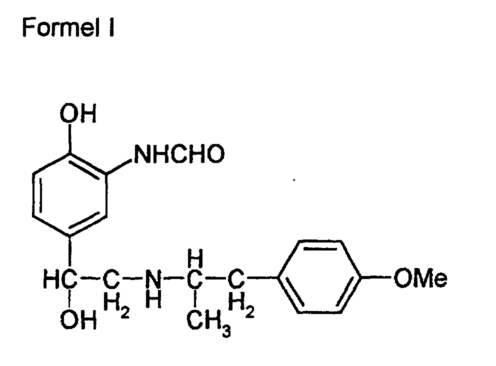

In der Vergangenheit hat sich herausgestellt, daß flüssige Aerosolformulierungen von Formoterol zur Verwendung in Inhalatoren für die ambulante inhalative Behandlung nicht geeignet sind, da Formoterol in Lösung nicht ausreichend stabil gelagert werden kann, um die pharmazeutische Qualität der Formulierung auch über einen längeren Zeitraum garantieren zu können. Deshalb wird Formoteol in der inhalativen Therapie bisher nur in Pulverform eingesetzt.

### Beschreibung der Erfindung

Die vorliegende Erfindung betrifft ein flüssiges Wirkstoffkonzentrat mit Formoterol in Form seiner freien Base oder in Form eines seiner pharmaokolgisch verträglichen Salze oder Additionsprodukte (Addukte) als Wirkstoff. Als Salz wird besonders das Formoterol-Fumarat, als Additionsprodukt ein Hydrat des Formoterols bevorzugt. Im weiteren Kontext dieser Beschreibung wird unter der Bezeichnung Formoterol sowohl die freie Base gemäß Formel I verstanden, als auch Formoterol-Salze und sonstige Additionsprodukte, sofern es nicht anders gekennzeichnet oder eindeutig aus dem Zusammenhang zu entnehmen ist.

Das erfidnungsgemäße Wirkstoffkonzentrat kann durch Verdünnen mit einer pharmakoloigsch verträglichen Flüssigkeit, die gegebenenfalls pharmazeutische Hilfs- und Zusatzstoffe enthält, in eine Arzneimittelzubereitung (Aerosolformulierung) überführt werden, die unter Zuhilfenahme eines Verneblers in ein inhalierbares Aerosol überführt wird.
Daher betrifft die Erfindung auch die Verwendung eines solchen Wirkstoffkonzentrats in der Inhalationstherapie.

Das erfindungsgemäße Wirkstoffkonzentrat betrifft Lösungen oder Suspensionen, in denen Formoterol hochkonzentriert in einem pharmakologisch geeigneten Flüssigkeit gelöst oder suspendiert vorliegt und die sich dadurch auszeichnen, daß darin der Wirkstoff Formoterol über einen Zeitraum von mehreren Monaten gegebenefalls bis zu mehreren Jahren gelagert werden kann, ohne daß die pharmazeutische Qualität beeinträchtigt wird.

Unter dem Begriff "Wirkstoffkonzentrat" wird eine Lösung oder Suspension eines Wirkstoffs verstanden, in der der Wirkstoff Formoterol hochkonzentriert in einer pharmakolgisch verträglichen Flüssigkeit als Lösung oder Suspension vorliegt. Bevorzugt sind Suspensionen, da diese sich als besonders lagerstabil erwiesen.

Unter "hochkonzentriert" wird eine Konzentration des Wirkstoffs verstanden, die üblicherweise zu hoch ist, um die entsprechende Lösung oder Suspension ohne Verdünnung in therapeutisch vertretbarer Weise für inhalative Zwecke einsetzen zu können. Erfindungsgemäß liegt die Formoterol-Konzentration in dem Wirkstoffkonzentrat zwischen 10 mg/ml und 500 mg/ml. Bevorzugt liegt die Mindestkonzentration bei wenigstens 75 mg/ml. Bevorzugte Konzentrationen liegen zwischen 100 mg/ml bis 400 mg/ml, insbesondere zwischen 250 mg/ml bis 350 mg/ml. Die Konzentrationsangaben beziehen sich auf mg freie Base Formoterol pro ml Wirkstoffkonzentrat. Im Fall von Formoterolsalzen oder dessen Additionsverbindungen sind die Konzentrationsangaben entsprechend der freien Base umzurechnen.

Unter dem Begriff "pharmakologisch geeignete Flüssigkeit" wird im Sinn der vorliegenden Erfindung ein Lösungs- oder Suspensionsmittel verstanden, das kein verflüssigtes Treibgas ist. Bevorzugt sind polare Flüssigkeiten, insbesondere bevorzugt sind protische Flüssigkeiten.

Beispiele für polare Lösungs- oder Suspensionsmittel sind z.B. Dimethylsulfoxid oder Verbindungen, die Hydroxylgruppen oder andere polare Gruppen enthalten, beispielsweise Wasser oder Alkohole - insbesondere Ethanol, Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester u.ä..

Beispiele für protische Flüssigkeiten, die im Kontext der Erfindung die am stärksten bevorzugten Lösungs- bzw. Suspensionsmittel sind, sind Wasser, wäßrige Salzlösungen mit einem oder mehreren pharmakologisch verträglichen Salz(en), Ethanol oder einem Gemisch daraus.
Im Fall von wäßrigen Ethanol- Gemischen liegt das Volumenverhältnis Ethanol zu Wasser bzw. zur wäßrigen Salzlösung zwischen 5:95 und 99:1, bevorzugt zwischen 40:60 und 96:4, insbesondere bevorzugt zwischen 75:25 und 96:4. Ein besonders bevorzugtes Verhältnis liegt zwischen 40:60 und 60:40.

Für eine Salzlösung als Lösungs- oder Suspensionsmittel oder als Bestandteil davon besonders gut geeignete Salze sind solche die nach Applikation keine oder nur eine vernachlässigbar geringe pharmakologische Wirkung entfalten.

Salzlösungen werden bevorzugt bei Suspensionskonzentraten eingesetzt. Durch den Salzzusatz wird das Lösungsvermögen von Wasser für Formoterol deutlich reduziert, so daß ein die suspendierten Teilchen stabilisierender Effekt erzielt wird. Gegebenenfalls können gesättigte Salzlösungen verwendet werden. Die Menge an Salz hängt dabei von der exakten Zusammensetzung des Lösungs- oder Suspensionsmittels ab und seinem Vermögen den Wirkstoff zu lösen. In wäßrigen Suspensionen im Sinn des erfindungsgemäßen Wirkstoffkonzentrats sollte Formoterol zu weniger als 0,5% gelöst vorliegen, bevorzugt zu weniger als 0,1 %, wobei sich die Angaben auf die Gesamtmenge (Gewicht) an Formoterol beziehen. Liegt die gelöste Menge dennoch über den angegebenen Werten, kann es durch Zugabe von Salz unterhalb dieser Werte gesenkt werden. In der Regel kann die Löslichkeit durch Salzzugabe auf die Hälfte gesenkt werden, in manchen Fällen auf ein Fünftel oder noch niedriger.
Bevorzugt sind Salzlösungen mit einem Salzgehalt von bis zu 50 Gew%, insbesondere bevorzugt bis zu 20 Gew.%.
Als Salze können sowohl anorganische als auch organische Salze verwendet werden. Bevorzugt sind anorganische Salze wie Natriumchlorid, Alkali- oder Ammonium-Halogensalze. Besonders bevorzugt ist Natriumchlorid. Als organische Salze eignen sich beispielsweise die Natrium-, Kalium- oder Ammoniumsalze der folgenden Säuren: Ascorbinsäure, Zitronensäure, Apfelsäure, Weinsäure, Maleinsäure, Bernsteinsäure, Fumarsäure, Essigsäure, Ameisensäure und/oder Propionsäure.

Dem Lösungs- oder Suspensionsmittel können Co-Solventien zugesetzt werden. Co-Solventien sind dazu geeignet, die Löslichkeit von Hilfsstoffen und gegebenenfalls des Formoterols zu erhöhen.

Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester, sofern sie nicht bereits das Lösungs- oder Suspensionsmittel sind.

Dem erfindungsgemäßen Wirkstoffkonzentrat können auch weitere Hilfs- und Zusatzstoffe zugesetzt werden.

Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Formoterol in dem pharmakologisch geeigneten Lösungs- oder Suspenionsmittel formuliert werden kann, um die qualitativen Eigenschaften des Wirkstoffkonzentrats oder der durch Verdünnung erhaltbaren, für inhalative Applikation geeigneten Arzneimittelzubereitung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe zur Stabilisierung von Suspensionen, sonstige Stabilisatoren, Komplexbildner, Antioxidantien und/oder Konservierungsstoffe, die die Verwendungsdauer der fertigen Arzneimittelformulierung verlängern, Geschmackstoffe, Vitamine, Antioxidantien und/oder sonstige dem Stand der Technik bekannte Zusatzstoffe.

Als oberflächenaktive Substanzen kann das Wirkstoffkonzentrat beispielsweise Sojalecithin, Ölsäure, Sorbitanester, wie Sorbitantrioleat oder andere aus dem Stand der Technik bekannte oberflächenaktive Stoffe (Surfactants) in der üblichen Konzentration enthalten.

Es hat sich gezeigt, daß der Zusatz einer organischen oder anorganischen Säure, bevorzugt in Kombination mit einem Komplexbildner, zu einer Verbesserung der Stabilität (Lagerstabilität) von Formoterolhaltigen Lösungen oder Suspensionen führt, insbesondere wenn diese Ethanol als Lösungsmittel enthalten.

Beispiele für diesbezüglich bevorzugte anorganische Säuren sind: Salzsäure, Salpetersäure, Schwefelsäure und/oder Phosphorsäure.
Beispiele für besonders geeignete organische Säuren sind: Ascorbinsäure, Zitronensäure, Apfelsäure, Weinsäure, Maleinsäure, Bersteinsäure, Fumarsäure, Essigsäure, Ameisensäure und/oder Propionsäure u. andere. Bevorzugte Säuren sind Salzsäure und/oder Fumarsäure

Die Konzentration der Säure wird so gewählt, daß das Wirkstoffkonzentrat einen pH-Wert von 2,0 bis 7,0, bevorzugt zwischen 4,0 und 6,0 und ganz bevorzugt zwischen 4,5 und 5,5 aufweist.

Als Komplexbildner können beispielsweise EDTA (Ethylendiamintetraessigsäure, bzw. ein Salz davon, wie beispielsweise das Dinatriumsalz), Zitronensäure, Nitrilotriessigsäure und deren Salze eingesetzt werden. Bevorzugt ist EDTA.

Konservierungsstoffe können eingesetzt werden, um das Konzentrat vor Kontamination mit pathogenen Keimen zu schützen. Als Konservierungsstoffe eigenen sich die dem Stand der Technik bekannten, insbesondere Benzalkoniumchlorid oder Benzoesäure bzw. Benzoate wie Natriumbenzoat.

Als Antioxidantien eignen sich die bekannten pharmakologisch verträglichen Antioxidantien, besonders Vitamine oder Provitamine wie sie im menschlichen Organismus vorkommen, beispielsweise Ascorbinsäure oder Vitamin E.

Liegt das Formoterol in dem erfindungsgemäßen Wirkstoffkonzentrat als Suspension vor, werden die Teilchen bevorzugt in einer Partikelgröße von bis zu 20 µm formuliert, bevorzugt bis zu 10 µm und insbesondere bevorzugt bis zu 5 µm.

Suspensionen sind als Wirkstoffkonzentrat am stärksten bevorzugt.

Gegebenenfalls kann das erfindungsgemäße Wirkstoffkonzentrat einen oder mehrere weitere(n) Wirkstoff(e) aus der Gruppe der Betamimetica, Anticholinergika, Antiallergika, Leukotrien-Antagonisten und insbesondere Steroide enthalten.

Das erfindungsgemäße Wirkstoffkonzentrat hat den Vorteil, daß Formoterol so formuliert werden kann, daß es über einen längeren Zeitraum stabil bleibt. Dabei ist es nicht notwendig, daß das Konzentrat abgesehen von der Wirkstoffkonzentration in seiner Zusammensetzung der fertigen Arzneimittelzubereitung entspricht. Zum Beispiel kann das Konzentrat in seinem pH-Wert erheblich von dem pH-Wert der zu applizierenden Arzneimittelzubereitung abweichen, wenn dadurch eine stabilere Lagerung von Formoterol möglich wird.

Das erfindungsgemäße Wirkstoffkonzentrat ist als solches üblicherweise nicht direkt zur medizinischen Verwendung, insbesondere zur inhalativen Applikation geeignet. Wie bereits geschildert, besteht eine Verwendung des Wirkstoffkonzentrats darin, es in eine Arzneimittelzubereitung (Aerosolformulierung) zu überführen. Dabei wird unter dem Begriff "Arzneimittelzubereitung" eine Formulierung eines Arzneimittels verstanden, die zur inhalativen Applikation geeignet ist und bei der ein Arzneimittel oder Arzneimittelgemisch in der notwendigen und / oder empfohlenen Konzentration appliziert werden kann (können).

Die Arzneimittelzubereitung ist bevorzugt derart, daß sie die mittels eines geeigneten Verneblers inhalativ applizierbar ist.

Eine bevorzugte Maßnahme zur Überführung des Wirkstoffkonzentrats in eine applizierbare Arzneimittelzubereitung, stellt das Verdünnen des erfindungsgemäßen Wirkstoffkonzentrats durch ein pharmakologisch geeignetes Lösungs- oder Suspensionsmittel dar.

Um die zu applizierende Formulierung zu erhalten, wird das Formoterol-Wirkstoffkonzentrat auf beispielsweise 0,9 mg/ml bis 1,5 mg/ml mit dem Verdünnungsmittel verdünnt.

Zum Zweck der Verdünnung bevorzugte Lösungs- oder Suspensionsmittel sind treibmittelfreie Flüssigkeiten, bevorzugt polare, insbesondere bevorzugt protische Flüssigkeiten. An dieser Stelle sei angemerkt, daß die einzelnen Komponenten oder Inhaltsstoffe des Verdünnungsmittels so definiert sind, wie im Zusammenhang mit dem Wirkstoffkonzentrat angegeben, sofern diese Komponenten oder Inhaltsstoffe dort beschrieben wurden oder sofern es nicht anders gekennzeichnet ist.

Besonders bevorzugte Verdünnungsmittel sind Wasser, wäßrige Salzlösungen mit einem oder mehreren pharmakologisch verträglichen Salz(en), Ethanol oder einem Gemisch daraus. Im Fall von wäßrigen Ethanol- Gemischen liegt das Volumenverhältnis Ethanol zu Wasser bzw. zur wäßrigen Salzlösung zwischen 5:95 und 99:1, bevorzugt zwischen 40:60 und 96:4, insbesondere bevorzugt zwischen 75:25 und 96:4. Ein besonders bevorzugtes Verhältnis liegt zwischen 40:60 und 60:40.

Es ist weder selbstverständlich noch notwendig, daß das Verdünnungsmittel identisch mit dem Lösungs- oder Suspensionmittel des Wirkstoffkonzentrats ist. Gegebenenfalls kann letzteres auch nur einen oder wenige Bestandteile des Verdünnungsmittels aufweisen.

Es sei an dieser Stelle ausdrücklich darauf hingewiesen, daß die bereits im Zusammenhang mit dem erfindungsgemäßen Wirkstoffkonzentrat erwähnten Co-Solventien und/oder Hilfs- oder Zusatzstoffe und/oder weiteren Wirkstoffe auch oder nur in dem Verdünnungsmittel gelöst oder suspendiert vorliegen können.

Bevorzugte Ausführungsformen des Verdünnungsmittels enthalten Konservierungsmittel und/oder Komplexbildner.

Gegebenenfalls kann in dem Verdünnungsmittel eine Puffersubstanz, wie z.B. Trinatriumphosphat, Dinatriumhydrogenphosphat, Natriumdihydrogenphosphat, Na-EDTA, EDTA, Gemische daraus u.a. aus dem Stand der Technik bekannte Stoffe vorhanden sein. Bevorzugt sind Natriumdihydrogenphosphat, Dinatriumhydrogenphosphat, Trinatriumhydrogenphosphat, Kaliumdihydrogenphosphat, Kaliumhydrogenphosphat, Trikaliumhydrogenphosphat und Gemische daraus. Puffersubstanzen sind besonders dann von Vorteil, wenn das erfindungsgemäße lagerfähige Wirkstoffkonzentrat einen pH-Wert aufweist, der deutlich von dem für die Applikation gewünschten abweicht, z.B. wenn dies die Stabilität des Wirkstoffs während der Lagerung erhöht. In diesem Fall liegt die Puffersubstanz im Verdünnungsmittel in einer solchen Konzentration vor, daß nach Vermischen des Wirkstoffkonzentrats mit dem Verdünnungsmittel eine applikationsfähige Aerosolformulierung mit dem gewünschten pH-Wert, bevorzugt zwischen 2,0 und 7,0, besonders bevorzugt zwischen 4,0 und 6,0 und ganz besonders bevorzugt zwischen 4,5 und 5,5 vorliegt.

In einer bevorzugten Ausführungsform enthält die Arzneimittelzubereitung einen Komplexbildner, der bevorzugt aus einem im Zusammenhang mit dem Wirkstoffkonzentrat genannten Komplexbildner ausgewählt wird. Die Menge an Komplexbildner beträgt bis zu 100 mg/100 ml bevorzugt bis zu 50 mg /100ml. Bevorzugter Komplexbildner ist EDTA.

Die zu applizierende Arzneimittelzubereitung bestimmt zusammen mit dem Wirkstoffkonzentrat die genaue Zusammensetzung des Verdünnungsmittels.

Weder das erfindungsgemäße lagerfähige Wirkstoffkonzentrat noch die durch Verdünnung erhaltene zu applizierende Arzneimittelzubereitung enthält ein Treibmittel.

Bevorzugt findet das Vermischen bei Umgebungstemperatur und Normaldruck statt. Ein Vorteil des erfindungsgemäßen Wirkstoffkonzentrats besteht darin, daß es sich durch Verdünnen innerhalb von sehr kurzer Zeit, beispielsweise innerhalb weniger Minuten oder gegebenenfalls innerhalb von wenigen Sekunden in eine therapeutisch wirksame und/oder zur Verwendung in einem Vernebler geeignete Formulierung überführen läßt. Dabei kann das Vermischen auch von Patienten, durchgeführt werden, die in der Regel kein pharmazeutisches Wissen besitzen.

Zur Verwendung in der Inhalationstherapie wird das erfindungsgemäße Wirkstoffkonzentrat bevorzugt vor der ersten Applikation mittels eines geeigneten Verneblers verdünnt und die erhaltende Arzneimittelzubereitung wird dann durch den Vernebler zersteubt.

In diesem Kontext geeignete Vernebler sind solche, die nicht treibmittelhaltige flüssige Formulierungen vernebeln können. Bevorzugte Vernebler sind beispielsweise Inhalatoren oder Hochdruckzersteuber, wie sie in der WO91/14468 "Atomizing Device and Methods" oder der WO 97/12687, dort besonders die durch die Figuren 6a und 6b beschriebenen, offenbart werden, auf die hiermit in ihrer Gesamtheit Bezug genommen wird. In solchen Verneblern sind in der Regel zur Applikation bestimmte Arzneimittelzubereitungen, die als Lösung vorliegen gegenüber Suspensionen bevorzugt.

Bevorzugt werden das erfindungsgemäße Wirkstoffkonzentrat und das Verdünnungsmittel getrennt in einem für Inhalatoren geeignetem Behälter gelagert, der so beschaffen ist, daß die beiden Komponenten automatisch mit dem einsetzen des Behälters in den Vemebler oder unmittelbar vor der ersten Applikation - quasi *in situ -* miteinander vermischt werden. In diesem Zusammenhang bevorzugte Behälter, werden beispielsweise in der PCT/EP 95/03183, in der WO 97/39831 und dort besonders durch die Figuren 1, 2, 2a oder 3b oder in der deutschen Patentanmeldung mit dem Aktenzeichen 198 47 968.9 und dort besonders durch die Figuren 1 bis 11, besonders Figur 3, beschriebenen Kartuschen offenbart, auf die hiermit in ihrer Gesamtheit Bezug genommen wird. Diese Behälter sind insbesondere zur Verwendung in einem Hochdruckzersteuber der oben beschriebenen Art geeignet.
In einem solchen Behälter können zwei oder mehr voneinander getrennte Kammern ausgebildet sind, wobei in wenigstens einer der Kammern das erfindungsgemäße Wirkstoffkonzentrat und in einer anderen Kammer das Verdünnungsmittel gelagert wird. Der Behälter ist so beschaffen, daß die beiden getrennt gelagerten Komponenten bereits dadurch miteinander vermischt werden können, daß der Behälter in den bestimmungsgemäßen Inhalator eingesetzt wird. Dabei ist die Menge der beiden Komponenten so dimensioniert, daß nach Vermischung der beiden Komponenten eine Aerosolformulierung entsteht, in der der oder die Wirkstoff(e) so konzentriert vorliegen (vorliegt), daß durch eine einmalige oder einige wenige Anwendungen des entsprechenden Verneblers die empfohlenen therapeutische Einzeldosis verabreicht werden kann. Im Rahmen der vorliegenden Beschreibung kann ein solches oder ein ähnliches Verfahren zur Herstellung der zu applizierendes Aerosolformulierung als *"in situ"* bzw. "quasi *in situ"* bezeichnet werden, wenn für den Benutzer keine Handgriffe verbunden sind, die über die normalen Handgriffe zur Inbetriebnahme eines Inhalators und der Verwendung der Aerosolformulierung mittels des Inhalators hinaus- oder vorausgehen.

Zum Zweck der Lagerung in der oben erwähnten Kartusche wird in bevorzugten Ausführungsformen die Menge des erfindungsgemäßen, lagerfähigen Wirkstoffkonzentrats so gewählt, daß sie einem Volumen von 0,001 bis zu ca. 0,05 ml, bevorzugt von 0,001 bis zu 0,02 ml entspricht.

Neben den beschriebenen können auch andere Behältern zur Lagerung der erfindungsgemäßen Formulierung benutzt werden.

Natürlich kann die Verdünnung mit einem pharmakologisch verträglichen Verdünnungsmittel auch anders vorgenommen werden, z.B. dadurch, daß das Verdünnungsmittel in einem offenen Gefäß mit dem Wirkungskonzentrat vermischt wird oder auf eine andere Art und Weise.

### Beispiele

### Beispiel 1

5 mg Formoterol (Teilchengröße: 5 µm) werden als Suspension mit 0,015 ml Wasser zur Lagerung formuliert. Durch Fumarsäure wird ein pH-Wert von 5,0 eingestellt.

Herstellung der inhalativ applizierbaren Arzneimittelzubereitung:
Zur inhalativen Anwendung wird die Suspension mit 4,5 ml einer 1:1 Lösung Wasser/Ethanol (v/v) verdünnt, wobei die Verdünnungslösung 0,45 mg Benzalkoniumchlorid und 2,25 mg Na- EDTA enthält und mit HCI auf einen pH-Wert von 5,0 eingestellt ist.

Die Konzentration des Wirkstoffkonzentrats liegt um das ca. 300-fache über der Konzentration der zu applizierenden Lösung.

### Beispiel 2

5 mg Formoterol (Teilchengröße: 5 µm) werden mit 0,015 ml einer 20Gew% wäßrigen NaCI-Lösung als Suspension zur Lagerung formuliert. Durch Fumarsäure wird ein pH-Wert von 5,0 eingestellt.

Herstellung der inhalativ applizierbaren Arzneimittelzubereitung:
Zur inhalativen Anwendung wird die Suspension mit 4,5 ml einer 1:1 Lösung Wasser/Ethanol (v/v) verdünnt, wobei die Verdünnungslösung 0,45 mg Benzalkoniumchlorid und 2,25 mg Na- EDTA enthält und mit HCI auf einen pH-Wert von 5,0 eingestellt ist.

Die Konzentration des Wirkstoffkonzentrats liegt um das ca. 300-fache über der Konzentration der zu applizierenden Lösung.

### Beispiel 3

In einer wäßrigen Lösung mit einem pH-Wert von 5,0, zersetzt sich Formoterol bei 40°C innerhalb von 3 Monaten zu 10%. In einer vergleichbaren Suspension kann nach 6 Monaten Lagerung bei 40°C keinerlei Zersetzung beobachtet werden.

## Patentansprüche

1. Lagerfähiges treibmittel-freies Wirkstoffkonzentrat zur Herstellung einer treibmittel-freien Aerosolformulierung enthaltend Formoterol in Form seiner freien Base, eines seiner pharmakologisch verträglichen Salze oder eines seiner Additionsprodukte als Wirkstoff in einem pharmakologisch verträglichen Lösungsoder Suspensionsmittel, wobei die Konzentration an Formoterol zwischen 75 mg/ml und 500 mg/ml liegt.

2. Wirkstoffkonzentrat nach Anspruch 1, **dadurch gekennzeichnet, daß** die Konzentration an Formoterol zwischen 100 mg/ml und 400 mg/ml liegt.

3. Wirkstoffkonzentrat nach Anspruch 1, **dadurch gekennzeichnet, daß** die Konzentration an Formoterol zwischen 250 mg/ml und 350 mg/ml liegt.

4. Wirkstoffkonzentrat nach einem der vorangegangen Ansprüche, **dadurch gekennzeichnet, daß** das Lösungs- oder Suspensionsmittel ein polare, bevorzugt eine protische Flüssigkeit ist.

5. Wirkstoffkonzentrat nach einem der vorangegangen Ansprüche, **dadurch gekennzeichnet, daß** das Lösungs- oder Suspensionsmittel Wasser, eine wäßrige Salzlösung, bevorzugt eine Natriumchloridlöung, Ethanol oder ein Gemisch daraus ist.

6. Wirkstoffkonzentrat nach einem der vorangegangenen Ansprüche 1 bis 5, **dadurch gekennzeichnet, daß** das Formoterol gelöst ist.

7. Wirkstoffkonzentrat nach einem der vorangegangenen Ansprüche 1 bis 5, **dadurch gekennzeichnet, daß** das Formoterol suspendiert ist.

8. Wirkstoffkonzentrat nach einem der vorangegangenen Ansprüche 1 bis 5, **dadurch gekennzeichnet, daß** das Formoterol in Wasser oder einer wäßrigen Salzlösung suspendiert ist.

9. Wirkstoffkonzentrat nach einem der vorangegangenen Ansprüche 1 bis 5,
**dadurch gekennzeichnet, daß** die Formulierung eine oberflächenaktive Substanz, wie z.B. Sorbitanester, bevorzugt Sorbitantrioleat, Ölsäure und / oder Lecithin enthält.

10. Wirkstoffkonzentrat nach einem der vorangegangenen Ansprüche 1 bis 9,
**dadurch gekennzeichnet, daß** das Wirkstoffkonzentrat eine pharmakologisch verträgliche Säure, wie z.B. Salzsäure, Salpetersäure, Schwefelsäure, Phosphorsäure, Ascorbinsäure, Zitronensäure, Apfelsäure, Weinsäure, Maleinsäure, Fumarsäure, Bersteinsäure, Essigsäure, Ameisensäure und / oder Propionsäure, bevorzugt Salzsäure und/oder Fumarsäure, enthält.

11. Wirkstoffkonzentrat nach einem der vorangegangenen Ansprüche 1 bis 10, **dadurch gekennzeichnet, daß** der pH-Wert des Wirkstoffkonzentrats zwischen 2,0 und 7,0, bevorzugt zwischen 4,0 und 6,0 und insbesondere bevorzugt zwischen 4,5 und 5,5 liegt.

12. Wirkstoffkonzentrat nach einem der vorangegangenen Ansprüche 1 bis 11, **dadurch gekennzeichnet, daß** das Wirkstoffkonzentrat ein Konservierungsmittel, Antioxidans und / oder einen Komplexbildner enthält.

13. Wirkstoffkonzentrat nach einem der vorangegangenen Ansprüche 1 bis 12, **dadurch gekennzeichnet, daß** die Formulierung einen oder mehrere weitere(n) inhalativ wirksame(n) Wirkstoff(e), bevorzugt aus der Gruppe Betamimetica, Anticholinergika, Antiallergika, Leukotrien-Antagonisten und/oder Steroide enthält.

14. Lagerfähiges Wirkstoffkonzentrat mit Formoterol als Wirkstoff, **dadurch gekennzeichnet, daß** Formoterol als Suspension in Wasser in einer Konzentration zwischen 250 mg/ml und 350 mg/ml vorliegt und daß das Wirkstoffkonzentrat auf einen pH-Wert von 4,5 bis 5,5 eingestellt ist und gegebenenfalls ein pharmakologisch verträgliches Salz in einer Menge enthält, daß Formoterol zu weniger als 0,5 Gew.%, bevorzugt weniger als 0,1 Gew.% gelöst vorliegt.

15. Verwendung eines Formoterol-Wirkstoffkonzentrats gemäß einem der vorangegangenen Ansprüche 1 bis 14 zur inhalativen Applikation, **dadurch gekennzeichnet, daß** die Formulierung vor der Applikation mit einem treibgasfreien, pharmakologisch verträglichen Verdünnungsmittel so verdünnt wird, daß die Konzentration an Formoterol zwischen 0,9 und 1,5 mg/ml liegt.

16. Verwendung eines Formoterol-Wirkstoffkonzentrats nach Anspruch 15, **dadurch gekennzeichnet, daß** das Verdünnungsmittel Wasser, eine wäßrige Salzlösung, Ethanol oder ein Gemisch daraus ist.

17. Verwendung eines Formoterol-Wirkstoffkonzentrats gemäß Anspruch 15, **dadurch gekennzeichnet, daß** die Formoterol-Formulierung eine Formulierung gemäß Anspruch 14 ist und das Verdünnungsmittel ein Wasser-Ethanol-Gemisch ist, das ein Konservierungsmittel und Na-EDTA enthält und auf einen pH-Wert zwischen 4,5 und 5,5 eingestellt ist.

18. Lösungsformulierung zur inhalativen Therapie enthaltend ein Lösungsmittelgemisch aus Ethanol/Wasser, das auf einen pH-Wert von ca. 4,5 bis 5,5 eingestellt ist, gelöstes Formoterol in einer Konzentration von ca. 0,9 bis 1,5 mg/ml bezogen auf Formoterol, ein Konservierungsmittel in einer pharmakologisch verträglichen Menge und Na-EDTA in einer pharmakologisch verträglichen Menge.

19. Verwendung eines Formoterol-Wirkstoffkonzentrats gemäß einem der Ansprüche 1 bis 14 zur Herstellung einer Formoterol-haltige Aerosolformulierung für die inhalative Anwendung.

20. Verwendung eines Formoterol-Wirkstoffkonzentrats nach einem der vorangegangenen Ansprüche 1 bis 14 und 18 in Inhalatoren zur inhalativen Therapie.

## Claims

1. Propellant-free active substance concentrate suitable for storage for preparing a propellant-free aerosol formulation containing formoterol in the form of its free base, one of the pharmacologically acceptable salts thereof or one of the addition products thereof as the active substance, in a pharmacologically acceptable solvent or suspension agent, wherein the concentration of formoterol is between 75 mg/ml and 500 mg/ml.

2. Active substance concentrate according to claim 1, **characterised in that** the concentration of formoterol is between 100 mg/ml and 400 mg/ml.

3. Active substance concentrate according to claim 1, **characterised in that** the concentration of formoterol is between 250 mg/ml and 350 mg/ml.

4. Active substance concentrate according to one of the preceding claims, **characterised in that** the solvent or suspension agent is a polar, preferably a protic liquid.

5. Active substance concentrate according to one of the preceding claims, **characterised in that** the solvent or suspension agent is water, an aqueous saline solution, preferably a sodium chloride solution, ethanol or a mixture thereof.

6. Active substance concentrate according to one of the preceding claims 1 to 5, **characterised in that** the formoterol is dissolved.

7. Active substance concentrate according to one of the preceding claims 1 to 5, **characterised in that** the formoterol is suspended.

8. Active substance concentrate according to one of the preceding claims 1 to 5, **characterised in that** the formoterol is suspended in water or an aqueous saline solution.

9. Active substance concentrate according to one of the preceding claims 1 to 5, **characterised in that** the formulation contains a surfactant such as sorbitan ester, preferably sorbitan trioleate, oleic acid and/or lecithin.

10. Active substance concentrate according to one of the preceding claims 1 to 9, **characterised in that** the active substance concentrate contains a pharmacologically acceptable acid such as e.g. hydrochloric acid, nitric acid, sulphuric acid, phosphoric acid, ascorbic acid, citric acid, malic acid, tartaric acid, maleic acid, fumaric acid, succinic acid, acetic acid, formic acid and/or propionic acid, preferably hydrochloric acid and/or fumaric acid.

11. Active substance concentrate according to one of the preceding claims 1 to 10, **characterised in that** the pH of the active substance concentrate is between 2.0 and 7.0, preferably between 4.0 and 6.0 and most preferably between 4.5 and 5.5.

12. Active substance concentrate according to one of the preceding claims 1 to 11, **characterised in that** the active substance concentrate contains a preservative, antioxidant and/or a complexing agent.

13. Active substance concentrate according to one of the preceding claims 1 to 12, **characterised in that** the formulation contains one or more additional inhalatively active pharmaceutical substances, preferably selected from the group comprising the betamimetics, anticholinergics, antiallergics, leukotriene antagonists and/or steroids.

14. Active substance concentrate suitable for storage, containing formoterol as active substance, **characterised in that** formoterol is present as a suspension in water in a concentration of between 250 mg/ml and 350 mg/ml and **in that** the active substance concentrate is adjusted to a pH of 4.5 to 5.5 and optionally contains a pharmacologically acceptable salt in an amount such that formoterol is present in solution at less than 0.5 % (w/w), preferably less than 0.1% (w/w).

15. Use of a formoterol active substance concentrate according to one of the preceding claims 1 to 14 for administration by inhalation, **characterised in that** the formulation is diluted before administration with a propellant-free, pharmacologically acceptable diluent so that the concentration of formoterol is between 0.9 and 1.5 mg/ml.

16. Use of a formoterol active substance concentrate according to claim 15, **characterised in that** the diluent is water, an aqueous saline solution, ethanol or a mixture thereof.

17. Use of a formoterol active substance concentrate according to claim 15, **characterised in that** the formoterol formulation is a formulation according to claim 14 and the diluent is a water/ethanol mixture which contains a preservative and Na-EDTA and is adjusted to a pH of between 4.5 and 5.5.

18. Formulation of a solution for inhalation therapy, containing a solvent mixture of ethanol/water which is adjusted to a pH of about 4.5 to 5.5, dissolved formoterol in a concentration of about 0.9 to 1.5 mg/ml based on formoterol, a preservative in a pharmacologically acceptable amount and Na-EDTA in a pharmacologically acceptable amount.

19. Use of a formoterol active substance concentrate according to one of claims 1 to 14 for preparing a formoterol-containing aerosol formulation for administration by inhalation.

20. Use of a formoterol active substance concentrate according to one of the preceding claims 1 to 14 and 18 in inhalators for inhalation therapy.

## Revendications

1. Concentré stable de principe actif sans propulseur pour la production d'une formulation d'aérosol sans propulseur contenant du formotérol sous forme de sa base libre, de l'un de ses sels pharmacologiquement acceptables ou de l'un de ses produits d'addition comme principe actif dans un solvant ou agent de suspension pharmacologiquement acceptable, où la concentration en formotérol est située entre 75 mg/ml et 500 mg/ml.

2. Concentré de principe actif selon la revendication 1 **caractérisé en ce que** la concentration en formotérol est située entre 100 mg/ml et 400 mg/ml.

3. Concentré de principe actif selon la revendication 1 **caractérisé en ce que** la concentration en formotérol est située entre 250 mg/ml et 350 mg/ml.

4. Concentré de principe actif selon l'une des revendications précédentes **caractérisé en ce que** le solvant ou agent de suspension est un liquide polaire, de préférence un liquide protique.

5. Concentré de principe actif selon l'une des revendications précédentes **caractérisé en ce que** le solvant ou agent de suspension est l'eau, une solution aqueuse de sel, de préférence une solution de chlorure de sodium, l'éthanol ou un mélange de ceux-ci.

6. Concentré de principe actif selon l'une des revendications 1 à 5 précédentes **caractérisé en ce que** le formotérol est dissous.

7. Concentré de principe actif selon l'une des revendication 1 à 5 précédentes **caracterisé en ce que** le formoterol est en suspension.

8. Concentré de principe actif selon l'une des revendications 1 à 5 précédentes **caractérisé en ce que** le formotérol est en suspension dans l'eau ou dans une solution aqueuse de sel.

9. Concentré de principe actif selon l'une des revendications 1 à 5 précédentes **caractérisé en ce que** la formulation contient une substance tensioactive, comme un ester de sorbitan, par exemple, de préférence le trioléate de sorbitan, l'acide oléique et/ou une lécithine.

10. Concentré de principe actif selon l'une des revendications 1 à 9 précédentes **caractérisé en ce que** le concentré de principe actif contient un acide pharmacologiquement acceptable, comme l'acide chlorhydrique, l'acide nitrique, l'acide sulfurique, l'acide phosphorique, l'acide ascorbique, l'acide citrique, l'acide malique, l'acide tartrique, l'acide maléique, l'acide fumarique, l'acide succinique, l'acide acétique, l'acide formique et/ou l'acide propionique, par exemple, de préférence l'acide chlorhydrique et/ou l'acide fumarique.

11. Concentré de principe actif selon l'une des revendications 1 à 10 précédentes **caractérisé en ce que** le pH du concentré de principe actif est situé entre 2,0 et 7,0, de préférence entre 4,0 et 6,0 et de manière particulièrement préférée entre 4,5 et 5,5.

12. Concentré de principe actif selon l'une des revendications 1 à 11 précédentes **caractérisé en ce que** le concentré de principe actif contient un conservateur, un antioxydant et/ou un complexant.

13. Concentré de principe actif selon l'une des revendications 1 à 12 précédentes **caractérisé en ce que** la formulation contient un ou plusieurs autres principes actifs efficaces par inhalation, de préférence du groupe des bêta-mimétiques, des anticholinergiques, des anti-allergiques, des antagonistes des leucotriènes et/ou des stéroïdes.

14. Concentré stable de principe actif contenant du formotérol comme principe actif **caractérisé en ce que** le formotérol est présent sous forme de suspension dans l'eau en une concentration située entre 250 mg/ml et 350 mg/ml et **en ce que** le concentré de principe actif est ajusté à un pH de 4,5 à 5,5 et contient éventuellement un sel pharmacologiquement acceptable en une quantité telle que le formotérol est présent à l'état dissous à raison de moins de 0,5 % en masse, de préférence moins de 0,1 % en masse.

15. Utilisation d'un concentré de principe actif formotérol selon l'une des revendications 1 à 14 précédentes pour l'application par inhalation **caractérisée en ce que**, avant l'application, la formulation est diluée avec un diluant sans gaz propulseur, pharmacologiquement acceptable, de telle sorte que la concentration en formotérol est située entre 0,9 et 1,5 mg/ml.

16. Utilisation d'un concentré de principe actif formotérol selon la revendication 15 **caractérisée en ce que** le diluant est l'eau, une solution aqueuse de sel, l'éthanol ou un mélange de ceux-ci.

17. Utilisation d'un concentré de principe actif formotérol selon la revendication 15 **caractérisée en ce que** la formulation de formotérol est une formulation selon la revendication 14 et le diluant est un mélange eau-éthanol, qui contient un conservateur et Na-EDTA et est ajusté à un pH situé entre 4,5 et 5,5.

18. Formulation de solution pour la thérapie par inhalation contenant un mélange de solvants d'éthanol/eau qui est ajusté à un pH d'environ 4,5 à 5,5, du formotérol dissous en une concentration d'environ 0,9 à 1,5 mg/ml en formotérol, un conservateur en une quantité pharmacologiquement acceptable et du Na-EDTA en une quantité pharmacologiquement acceptable.

19. Utilisation d'un concentré de principe actif formotérol selon l'une des revendications 1 à 14 pour la production d'une formulation d'aérosol contenant du formotérol pour l'application par inhalation.

20. Utilisation d'un concentré de principe actif formotérol selon l'une des revendications 1 à 14 et 18 précédentes dans des inhalateurs pour la thérapie par inhalation.
